# EUROPÄISCHE PATENTSCHRIFT

(11) **EP 2 627 413 B1**
(45) Veröffentlichungstag und Bekanntmachung des Hinweises auf die Patenterteilung: **12.04.2017**
(21) Anmeldenummer: 11754417.1
(22) Anmeldetag: 09.09.2011
(51) Int. Cl.: A61Q 5/08, A61K 8/22, A61K 8/73

(54) **MITTEL ZUR OXIDATIVEN FARBVERÄNDERUNG VON KERATINHALTIGEN FASERN**
AGENTS FOR OXIDATIVELY CHANGING THE COLOUR OF KERATIN-CONTAINING FIBRES
PRODUIT PERMETTANT DE MODIFIER PAR OXYDATION LA COULEUR DE FIBRES CONTENANT DE LA KÉRATINE

(30) Priorität: 11.10.2010 DE 102010042252
(43) Veröffentlichungstag der Anmeldung: 21.08.2013
(73) Patentinhaber: Henkel AG & Co. KGaA, 40589 Düsseldorf (DE)
(72) Erfinder: GOUTSIS, Konstantin, 41812 Erkelenz (DE); JANßEN, Frank, 41470 Neuss (DE); FRANKE, Ina, 40211 Düsseldorf (DE)
(86) Internationale Anmeldenummer: PCT/EP2011/065602
(87) Internationale Veröffentlichungsnummer: WO 2012/048966

(56) Entgegenhaltungen:
- EP-A1- 2 191 812
- WO-A1-2005/067874
- DE-A1-102007 041 490
- GB-A- 466 172
- Anonymous: "CEKOL Carboxymethyl Cellulose (CMC)", CP Kelco , 5. Februar 2013 (2013-02-05), XP002691657, Gefunden im Internet: URL:http://www.cpkelco.com/market_pharmace uticals/prod-cekol.html [gefunden am 2013-02-05]

## Beschreibung

Die vorliegende Erfindung betrifft Mittel zur oxidativen Farbveränderung im Bereich der Kosmetik, die sich besonders zum Aufhellen keratinischer Fasern, insbesondere menschlicher Haare, eignen.

Die Veränderung von Form und Farbe der Haare stellt einen wichtigen Bereich der modernen Kosmetik dar. Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich.

Die in Blondiermitteln enthaltenen Oxidationsmittel sind in der Lage, die Haarfaser durch die oxidative Zerstörung des haareigenen Farbstoffes Melanin aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt.

Aus Stabilitätsgründen werden handelsübliche Blondiermittel gewöhnlich in zwei getrennt voneinander verpackten Zubereitungen angeboten, die unmittelbar vor der Anwendung zu einer fertigen Anwendungszubereitung vermischt werden. Üblicherweise bestehen handelsübliche Blondiermittel aus einer flüssigen Oxidationsmittelzubereitung und einem Pulver, das feste Oxidationsmittel enthält. Produkte mit weiteren Komponenten werden ebenfalls im Handel angeboten. Eine gute Mischbarkeit der verschiedenen Zubereitungen ist wesentlich für die einwandfreie Handhabung des anwendungsbereiten Blondiermittels. Verklumpungen führen dazu, dass nur ein Teil der eingesetzten Wirkstoffe die Faser erreichen kann und haben eine Schwächung des erwünschten Blondiereffekts zur Folge. Zusätzlich bewirkt eine nicht vollständige Homogenisierung Konzentrationsschwankungen der unterschiedlichen Wirkstoffe in der fertigen Anwendungszubereitung und folglich eine ungleichmäßige Blondierwirkung auf der Faser. Daher besteht weiterhin Bedarf an neuen Blondiermittelzubereitungen mit guter Vermischbarkeit und verbesserten Blondierergebnissen.

Des Weiteren spielt die Viskosität der Zubereitung eine wichtige Rolle. Die gute Verteilbarkeit der Zubereitung über die gesamte Haarlänge und das Verhindern des Abtropfens sind wesentliche Eigenschaften für eine optimale Handhabung des Entfärbemittels. Dabei erwartet der Kunde, dass das Mittel dickflüssig genug ist, um beim Auftragen und während der Einwirkzeit nicht auf Material in der Umgebung z.B. Kleidung oder Böden, zu tropfen, was unerwünschte Verfärbungen hervorrufen kann. Gleichzeitig muss jedoch eine ausreichende Fließfähigkeit des Mittels gewährleistet sein, um ein gleichmäßiges Auftragen und eine gute Verteilbarkeit des Mittels über die gesamte Haarlänge zu ermöglichen. Es stellt sich die Aufgabe, die Fließfähigkeit des Mittels an die Ansprüche der Kunden anzupassen, ohne die Stabilität der Wirkstoffe des Mittels, wie z.B. Wasserstoffperoxid, zu verschlechtern. In vielen Fällen kommen Blondiermittel zum Einsatz, die Emulsionen beinhalten. Emulsionen neigen dazu, ihre rheologischen Eigenschaften im Verlauf der Lagerung in Abhängigkeit von den Lagerbedingungen zu verändern, was Einfluss auf die o.g. Verteilbarkeit und Vermischbarkeit hat. Gele zeigen hingegen eine bessere Homogenität und eine längere Lagerstabilität. Deshalb besteht weiterhin Bedarf an neuen Blondiermittelzubereitungen mit guter Stabilität und verbesserter Viskosität.

Einen weiteren wichtigen Aspekt der Anwendung von Blondiermitteln stellt die Kontrolle des Aufhellvorgangs auf der Faser dar. Üblicherweise wird der Entfärbevorgang mindestens ein Mal während der Einwirkzeit vom Kunden kontrolliert. Handelsübliche Blondiermittel sind in anwendungsbereitem Zustand in der Regel weiße bis farbige, trübe Gele oder Emulsionen. Bei der Anwendung dieser Blondiermittel ist es zur Kontrolle des Entfärbevorgangs während der Einwirkzeit notwendig, das Mittel in einem oder mehreren Bereichen der Fasern abzutragen. Der Fortschritt der Farbveränderung kann so vom Kunden beurteilt werden. Wenn nötig muss die Anwendungsmischung anschließend wieder auf die entsprechenden Stellen auf der Faser aufgetragen werden, um den Aufhellprozess fortzusetzen. Der Vorgang muss gegebenenfalls für weitere Kontrollen wiederholt werden. Die Anwendung transparenter Anwendungsmischungen vereinfacht diesen Kontrollschritt erheblich. Ein Abtragen des Blondiermittels von der Faser ist nicht notwendig. Stattdessen ermöglicht die Transparenz der Anwendungsmischung eine direkte, visuelle Beurteilung des Entfärbevorgangss zu jedem beliebigen Zeitpunkt während der Einwirkzeit. Die Bereitstellung transparenter Blondiermittel führt folglich zu einer verbesserten Handhabung des Blondiermittels und zu einer Vereinfachung der Anwendung.

WO 2005/067874 A1 beschreibt Blondiermittel, welche eine Mischung aus einem Oxidationsmittel, mindestens einem Stabilisator, mindestens einem Polymerverdicker und Wasser oder ein wässriges Lösungsmittel enthalten. Als wünschenswerte Eigenschaften des Mittels gemäß dieser Erfindung sind "Transparenz" und "angedickte Konsistenz" angegeben (S. 2, Abs 1 und 2). In WO 2005/067874 A1 werden Polymerverdicker aus synthetischen Polymeren und Alkalimagnesiumsilikate beansprucht. Beispiel 13 (S.14) der oben genannten Anmeldung beschreibt ein Blondiermittel, das in der pulverförmigen Komponente neben einem Acrylsäurepolymer unter anderem Xanthan Gum beinhaltet. Allerdings scheint Xanthan Gum an dieser Stelle zufällig eingesetzt worden zu sein. Weder die Beschreibung noch die beanspruchte Erfindung der genannten Anmeldung lassen darauf schließen, dass die Eigenschaften von Xanthan Gum erfindungsrelevant seien, geschweige denn die Vorteile der Verwendung von natürlichen Polymeren in diesen Mitteln erkannt wurden. DE 10 2007 041 490 A1 offenbart Blondiermittel, die transparent sind und durch Vermischen zweier getrennt verpackten Zubereitungen erhalten werden, und zwar: a) ein Blondierpulver und b) eine flüssige Entwicklerdispersion, wobei das Blondierpulver vorzugsweise Persulfate enthält und die Entwicklerdispersion eine Wasserstoffperoxidlösung ist. Sowohl das Blondierpulver als auch die Entwicklerdispersion können Verdickungsmittel wie z. B. Xanthan Gum enthalten. Aufgabe der vorliegenden Erfindung war es, die Eigenschaften handelsüblicher Blondiermittel in Bezug auf ihre Mischbarkeit und Viskosität zu verbessern. Weiterhin war es erwünscht eine homogen blondierende, transparente Anwendungsmischung mit diesen Eigenschaften zu erhalten, um zusätzlich eine visuelle Kontrolle des Aufhellvorgangs ohne Entfernen der Zubereitung von der Faser zu ermöglichen.

Es hat sich gezeigt, dass die Zugabe eines natürlichen Polymers zu der Oxidationsmittelzubereitung zu einer fließfähigen Zubereitung führt, welche eine leichte Einarbeitung einer pulverförmigen Zubereitung und eine gute Homogenisierung der beiden Komponenten zu einer fertig vermischten Anwendungsmischung gestattet. Außerdem weist die erfindungsgemäße Zubereitung gute Eigenschaften bezüglich der Viskosität auf. Ein weiterer Vorteil des erfindungsgemäßen Blondiermittels ist seine Transparenz, die eine visuelle Kontrolle des Aufhellgrades der keratinischen Faser während des Aufhellprozesses ermöglicht.

Mittel zum Aufhellen keratinischer Fasern, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
i. Zubereitung (A) mindestens ein Persulfat enthält und
ii. Zubereitung (B) fließfähig ist und mindestens ein Oxidationsmittel enthält,
dadurch gekennzeichnet, dass Zubereitung (B) Xanthan enthält und Zubereitung (A) frei von Xanthan ist. Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Die Zubereitungen (A) sind bevorzugt pulverförmig. Dabei können Pulver aus festen Bestandteilen mit unterschiedlichen Korngrößen eingesetzt werden. Üblicherweise kann es bevorzugt sein, wenn die Pulver jedoch eine möglichst homogene Korngröße aufweisen, insbesondere um eine einheitliche Dispersion bzw. Auflösung der Pulver in den Zubereitungen (B) zu erleichtern.

Die Zubereitungen (A) können die Wirkstoffe in einem festen kosmetischen Träger enthalten. Ein fester kosmetischer Träger kann Salze der Kieselsäure, insbesondere Salze der Silicate und Metasilicate mit Ammonium, Alkalimetallen sowie Erdalkalimetallen enthalten. Insbesondere Metasilicate, die sich gemäß Formel (SiO₂)ₙ(M₂O)ₘ, wobei M für ein Ammoniumion, ein Alkalimetall oder ein halbes Stöchiometrieäquivalents eines Erdalkalimetalls steht, durch das Verhältnis zwischen n und m von ≤ 1 auszeichnen und sich als kettenförmige polymere Strukturen des Anions [SiO_{3]}²⁻ auffassen lassen, können bevorzugt eingesetzt werden. Natriummetasilicat der Formel [Na₂SiO₃]∞, ist dabei besonders bevorzugt. Erfindungsgemäß gleichfalls bevorzugt sind solche Silicate, die aus einem Silicat der Formel (SiO₂)ₙ(Na₂O)ₘ(K₂O)ₚ gebildet werden, wobei n für eine positive rationale Zahl und m und p unabhängig voneinander für eine positive rationale Zahl oder für 0 stehen, mit den Maßgaben, dass mindestens einer der Parameter m oder p von 0 verschieden ist und das Verhältnis zwischen n und der Summe aus m und p zwischen 2:1 und 4:1 liegt.

Weiterhin können die festen kosmetischen Träger so genannte Rieselhilfen enthalten, die ein Verklumpen oder Verbacken der Pulver-Bestandteile verhindern sollen. Als solche Rieselhilfen kommen bevorzugt wasserunlösliche, hydrophobierende oder Feuchtigkeit adsorbierende Pulver von Kieselgur, pyrogenen Kieselsäuren, Calciumphosphat, Calciumsilicaten, Aluminiumoxid, Magnesiumoxid, Magnesiumcarbonat, Zinkoxid, Stearaten, Fettaminen und dergleichen in Frage. Schließlich können die festen kosmetischen Träger noch zusätzlich ein Entstaubungsmittel enthalten, die die Staubbildung der pulverförmigen Bestandteile verhindert. Hierzu können insbesondere inerte Öle eingesetzt werden. Bevorzugt enthalten die festen, kosmetischen Träger als Entstaubungsmittel Esteröle oder Mineralöle, bevorzugt Kohlenwasseröle, wie flüssiges Paraffinöl.

Als ersten wesentlichen Inhaltsstoff enthält die Zubereitung (A) mindestens ein Persulfat. Erfindungsgemäß geeignete Persulfate sind anorganische Peroxoverbindungen. Diese sind bevorzugt ausgewählt aus Ammoniumperoxodisulfat, Alkalimetallperoxodisulfaten, Ammoniumperoxomonosulfat, Alkalimetallperoxomonosulfaten, Alkalimetallperoxodiphosphaten und/oder Erdalkalimetallperoxiden. Besonders bevorzugt sind Ammoniumperoxodisulfat und/oder Alkalimetallperoxodisulfate.

In einer bevorzugten Ausführungsform der vorliegenden Erfindung enthält Zubereitung (A) als Persulfat mindestens ein Peroxodisulfat-Salz, insbesondere ausgewählt aus Ammoniumperoxodisulfat und/oder Kaliumperoxodisulfat und/oder Natriumperoxodisulfat.

Weiterhin hat es sich bei den Arbeiten zur vorliegenden Erfindung als besonders bevorzugt erwiesen, wenn die Zubereitungen (A) mindestens zwei verschiedene Peroxodisulfate enthalten. Bevorzugte Peroxodisulfat-Salze sind dabei Kombinationen von Ammoniumperoxodisulfat mit Kaliumperoxodisulfat und/oder Natriumperoxodisulfat.

Die Zubereitungen (A) enthalten vorzugsweise Persulfat-Salze in einer Menge von 0,1 bis 80 Gew.-%, bevorzugt von 2 bis 50 Gew.-%, besonders bevorzugt von 3 bis 30 Gew.-% und insbesondere bevorzugt 5 bis 15 Gew.-% explizit 5, 6, 7, 8, 9, 10, 11, 12, 13, 14 oder 15 Gew.-% jeweils bezogen auf das Gesamtgewicht des Mittels.

Eine weitere Ausführungsform der vorliegenden Erfindung ist dadurch gekennzeichnet, dass die Zubereitung (A) frei von Xanthan ist. Unter "frei von" im Sinne der Erfindung sind Mengen von 0,01 Gew.-% und weniger, bezogen auf Zubereitung (A), zu verstehen.

Der erste Gegenstand der Erfindung umfasst Mittel zum Aufhellen keratinischer Fasern, die mindestens zwei getrennt voneinander verpackte Zubereitungen (A) und (B) sowie gegebenenfalls eine weitere getrennt von (A) und (B) verpackte Zubereitung (C) enthalten. Die Zubereitungen (B) und gegebenenfalls (C) enthalten die Wirkstoffe in einem fließfähigen, kosmetischen Träger. Die Grundlage des fließfähigen, kosmetischen Trägers ist dabei bevorzugt wässrig oder wässrigalkoholisch. Zum Zwecke der Haarbleiche sind solche Träger beispielsweise transparente Gele oder auch tensidhaltige schäumende Lösungen, wie beispielsweise Shampoos, Schaumaerosole oder andere Zubereitungen, die für die Anwendung auf dem Haar geeignet sind. Ein bevorzugter, fließfähiger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether enthalten. Bevorzugt sind dabei alle wasserlöslichen organischen Lösungsmittel.

Als wesentlichen Inhaltstoff enthält die Zubereitung (B) Xanthan. Wenn das Mittel zum Aufhellen keratinischer Fasern, genau zwei getrennt voneinander verpackte Zubereitungen (A) und (B) enthält, die unmittelbar vor der Anwendung zu einer Anwendungsmischung vermischt werden, so enthält Zubereitung (B) gemäß der Erfindung Xanthan. Wenn das Mittel zum Aufhellen keratinischer Fasern, mindestens drei getrennt voneinander verpackte Zubereitungen (A), (B) und (C) enthält, die unmittelbar vor der Anwendung zu einer Anwendungsmischung vermischt werden, so kann auch Zubereitung (C) Xanthan enthalten.

Xanthan ist kommerziell erhältlich wird beispielsweise unter den Handelsnamen Kelzan (Xanthan-Biopolymer, Kelco), Xanthan FN (Xanthan-Biopolymer, Jungbunzlauer), Keltrol z.B. Keltrol CG-T (Xanthan-Biopolymer, Kelco) oder Keltrol CG-SFT (Xanthan-Biopolymer, Kelco) angeboten.

In der Erfindung enthalten die Zubereitungen (B) und gegebenenfalls (C) Xanthan.

Erfindungsgemäß bevorzugt sind solche Xanthane, die nach der Quellung transparente Zubereitungen ergeben. Insbesondere bevorzugt ist die Verwendung des Xanthan-Biopolymers, welches unter dem Handelsnamen Keltrol CG-SFT der Firma Kelco vertrieben wird.

In einer bevorzugten Ausführungsform enthält Zubereitung (B) bezogen auf ihr Gewicht Xanthan in Mengen von 0,1 bis 10 Gew.-%, vorzugsweise von 0,5 bis 6 Gew.-%, besonders bevorzugt von 0,7 bis 5 Gew.-% und insbesondere bevorzugt von 1 bis 4 Gew.-%, explizit 1, 2, 3 oder 4 Gew.-%, wenn das Mittel zum Aufhellen keratinischer Fasern, genau zwei getrennt voneinander verpackte Zubereitungen (A) und (B) enthält, die unmittelbar vor der Anwendung zu einer Anwendungsmischung vermischt werden. Vorzugsweise enthalten die fertigen vermischten Anwendungszubereitungen bezogen auf ihr Gewicht, Xanthan in Mengen von 0,6 bis 5 Gew.-%, besonders bevorzugt von 1,0 bis 3,5 Gew.-% und insbesondere bevorzugt von 1,5 bis 2,5 Gew.-% explizit 1,5; 1,6; 1,7; 1,8; 1,9; 2,0; 2,1; 2,2; 2,3; 2,4 oder 2,5 Gew.-%.

Wenn das Mittel zum Aufhellen keratinischer Fasern, mindestens drei getrennt voneinander verpackte Zubereitungen (A), (B) und (C) enthält, die unmittelbar vor der Anwendung zu einer Anwendungsmischung vermischt werden, sind Zubereitungen bevorzugt, in denen Zubereitung (C) Xanthan enthält.

Es sind fertig vermischten Anwendungszubereitungen bevorzugt, die, bezogen auf das Gewicht der fertigen Anwendungszubereitung, Xanthan in Mengen von 0,6 bis 5 Gew.-%, besonders bevorzugt von 1,0 bis 3,5 Gew.-% und insbesondere bevorzugt 1,5 bis 2,5 Gew.-% explizit 1,5; 1,6; 1,7; 1,8; 1,9; 2,0; 2,1; 2,2; 2,3; 2,4 oder 2,5 Gew.-%.

In einer besonderen Ausführungsform enthalten die Zubereitungen (B) gemäß der Erfindung Wasserstoffperoxid als Oxidationsmittel.

Die Konzentration einer Wasserstoffperoxid-Lösung in der Oxidationsmittelzubereitung (B) wird einerseits von den gesetzlichen Vorgaben und andererseits von dem gewünschten Effekt bestimmt. Vorzugsweise enthalten die Zubereitungen (B) bezogen auf ihr Gewicht Wasserstoffperoxid in Mengen von 0,5 bis 30 Gew.-%, bevorzugt von 1 bis 20 Gew.-%, besonders bevorzugt von 5 bis 15 Gew.-% und insbesondere bevorzugt von 6 bis 12 Gew.-%, explizit 6, 7, 8, 9, 10, 11 oder 12 Gew.-%.

Erfindungsgemäß bevorzugte anwendungsbereite Mittel sind dadurch gekennzeichnet, dass sie, bezogen auf das Gesamtgewicht des anwendungsbereiten Mittels, 0,01 bis 12 Gew.-%, vorzugsweise 0,1 bis 10 Gew.-%, besonders bevorzugt 1 bis 8 Gew.-% Wasserstoffperoxid enthalten.

Zur Stabilisierung des Wasserstoffperoxid kann der pH-Wert der Zubereitung (B) bevorzugt auf pH 3 bis 5, besonders bevorzugt auf pH 3,5 bis 4,5 und insbesondere bevorzugt auf pH 3,8 bis 4,2 eingestellt werden.

Die viskosen Eigenschaften von Zubereitung (B) sind von Bedeutung für ihre gute Mischbarkeit und hohe Stabilität. In einer bevorzugten Ausführungsform sind die Zubereitungen (B) der vorliegenden Erfindung dadurch gekennzeichnet, dass sie eine Viskosität von 1.000 mPa·s bis 50.000 mPa·s, vorzugsweise von von 5.000 mPa·s bis 45.000 mPa·s und besonders bevorzugt von 7.000 mPa·s bis 40.000 mPa·s bei Messungen mit einem Rotationsviskosimeter von Brookfield, Spindelgröße 4, bei 25°C und 4 Upm, aufweisen. Die fertig angemischten und anwendungsbereiten Mittel weisen vorzugsweise eine Viskosität von 10.000 mPa·s bis 50.000 mPa·s und besonders bevorzugt von 18.000 mPa·s bis 30.000 mPa·s bei Messungen mit einem Rotationsviskosimeter von Brookfield, Spindelgröße 5, bei 25°C und 4 Upm auf.

Weiterhin ist die Einstellung des pH-Wertes von Bedeutung für gute Mischbarkeit und Stabilität. Erfindungsgemäß bevorzugt sind fertig angemischte und anwendungsbereite Mittel, deren pH-Wert zwischen 9 und 12 liegt.

Weiterhin kann es erfindungsgemäß von Vorteil sein, wenn Zubereitung (B) mindestens ein nichtionisches Tensid, bevorzugt mindestens einen ethoxylierten Fettalkohol mit 40 bis 60 Ethylenoxideinheiten enthält. Erfindungsgemäß ist darunter ein Anlagerungsprodukt von Ethylenoxid an einen Fettalkohol zu verstehen. Fettalkohole sind dabei gesättigte und ungesättigte Alkohole mit 12 bis 24 C-Atomen, welche linear oder verzweigt sein können. Die Molmenge an Ethylenoxid, die pro Mol Fettalkohol eingesetzt wurde, bezeichnet dabei den Ethoxylierungsgrad verstanden. Als nichtionisches Tensid eignen sich dabei insbesondere Ethylenoxid-Anlagerungsprodukte an Octylalkohol (Caprylalkohol), Nonylalkohol (Pelargonylalkohol), Undecylalkohol, Undec-10-en-1-ol, Dodecylalkohol (Laurylalkohol), 2,6,8-Trimethyl-4-nonanol (Isolaurylalkohol), Tridecylalkohol, Tetradecylalkohol (Myristylalkohol), Pentadecylalkohol, Hexadecylalkohol (Cetyl-/ Palmitylalkohol), Heptadecylalkohol, Octadecylalkohol (Stearylalkohol), Isostearylalkohol, (9Z)-Octadec-9-en-1-ol (Oleylalkohol), (9E)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenylalkohol), Nonadecan-1-ol (Nonadecylalkohol), Eicosan-1-ol (Eicosylalkohol / Arachylalkohol), (9*Z*)-Eicos-9-en-1-ol (Gadoleylalkohol), (5*Z*,8*Z*,11*Z*,14*Z*)-Eicosa-5,8,11,14-tetraen-1-ol (Arachidonalkohol), Heneicosylalkohol, Docosylalkohol (Behenylalkohol), (13*Z*)-Docos-13-en-1-ol (Erucylalkohol) oder (13*E*)-Docosen-1-ol (Brassidylalkohol). Es ist erfindungsgemäß ebenfalls möglich, Gemische von Fettalkoholen, die durch gezielte Mischung oder auch durch Gewinnungsverfahren als solche anfallen, einzusetzen. Beispiele sind Cocosalkohol (Mischung aus C₈-C₁₈-Fettalkoholen) oder Cetearylalkohol (1:1-Mischung aus C₁₆- und C₁₈-Fettalkoholen).

Bevorzugt sind Ethoxylierungsgrade von 20 bis 60. Erfindungsgemäß bevorzugte nichtionische Tenside vom Typ ethoxylierter Fettalkohol sind Ceteareth-20 und Ceteareth-50.

Weiterhin können die Blondiermittel Alkalisierungsmittel enthalten. Bevorzugte Alkalisierungsmittel sind beispielsweise Ammoniak, Alkanolamine, basischen Aminosäuren, sowie anorganischen Alkalisierungsmittel wie (Erd-)Alkalimetallhydroxide, (Erd-)Alkalimetallmetasilikate, (Erd-) Alkalimetallphosphate und (Erd-)Alkalimetallhydrogenphosphate. Als Metallionen dienen bevorzugt Lithium, Natrium und/oder Kalium. Insbesondere bevorzugtes Alkalisierungsmittel ist Ammoniak. Erfindungsgemäß einsetzbare, anorganische Alkalisierungsmittel sind bevorzugt ausgewählt aus Natriumhydroxid, Kaliumhydroxid, Calciumhydroxid, Bariumhydroxid, Natriumphosphat, Kaliumphosphat, Natriumsilicat, Kaliumsilicat, Magnesiumsilicat, Natriumcarbonat und Kaliumcarbonat. Besonders bevorzugt sind Natriumhydroxid und/oder Kaliumhydroxid.

Erfindungsgemäß einsetzbare Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Insbesondere bevorzugte Alkanolamine sind Monoethanolamin, 2-Amino-2-methyl-propanol und Triethanolamin.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, L-Ornithin, D- Ornithin, D/L- Ornithin, L-Histidin, D-Histidin und/oder D/L-Histidin. Besonders bevorzugt werden L-Arginin, D-Arginin und/oder D/L-Arginin als ein Alkalisierungsmittel im Sinne der Erfindung eingesetzt.

Manche Kunden empfinden die intensive Geruchsbildung von Ammoniak belästigend oder störend. Obwohl Ammoniak ein bevorzugtes Alkalisierungsmittel ist, können daher anwendungsbereite Zubereitungen erfindungsgemäß bevorzugt sein, die frei von Ammoniak sind. Bevorzugte Alkalisierungsmittel für Zubereitungen, die frei von Ammoniak sind, sind Monoethanolamin, 2-Amino-2-methyl-propanol und Triethanolamin.

Wenn die anwendungsbereiten Mischungen Alkalisierungsmittel enthalten, sind Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

In einer weiteren Ausführungsform der Erfindung, können Zubereitungen (A) und (B) mit weiteren separat verpackten Zubereitungen unmittelbar vor der Anwendung zu einer Anwendungsmischung vermischt werden.

In einer bevorzugten Ausführungsform der Erfindung enthält das erfindungsgemäße Mittel zusätzlich mindestens eine weitere getrennt von Zubereitungen (A) und (B) verpackte Zubereitung (C), wobei Zubereitung (C) mindestens ein Alkalisierungsmittel und mindestens ein natürliches Polymer enthält.

Bevorzugt enthält Zubereitung (C) natürliche Polymere. Als natürliches Polymer können beispielsweise Cellulosederivate verwendet werden, die als Verdickungsmittel Verwendung finden. Beispiele sind Agar-Agar, Carrageen, Alginate, Xanthan-Gum, Karaya-Gummi, Ghatti-Gummi, Tragant, Skleroglucangums oder Gummi arabicum, Alginate, Pektine, Polyosen, Guar-Gums, Johannisbrotbaumkernmehl, Leinsamengummen, Dextrane, Pektine, Staerke-Fraktionen und Derivate wie Amylose, Amylopektin und Dextrine, Gelatine und Casein sowie Cellulosederivate, wie beispielsweise Methylcellulose, Carboxyalkylcellulosen wie Carboxymethylcellulose und Hydroxyalkylcellulosen wie Hydroxyethylcellulose. Natürliche Polymere aus den genannten Substanzklassen sind kommerziell erhältlich und werden beispielsweise unter den Handelsnamen Deuteron<®>-XG (anionisches Heteropolysaccharid auf Basis von β-D-Glucose, D-Manose, D-Glucuronsaeure, Schoener GmbH), Deuteron<®>-XN (nichtionogenes Polysaccharid, Schoener GmbH), Protanal RF 6650 alginate (Natriumalginat, FMC Biopolymer), Cekol (Cellulose Gum, Kelco), Kelzan (Xanthan-Biopolymer, Kelco), Xanthan FN (Xanthan-Biopolymer, Jungbunzlauer), Keltrol z. B. Keltrol CG-T (Xanthan-Biopolymer, Kelco) oder Keltrol CG-SFT (Xanthan-Biopolymer, Kelco) angeboten. Erfindungsgemäß bevorzugt sind solche Alkalisierungsmittel, die bereits zuvor beschrieben wurden.

Wenn die Zubereitungen (C) Alkalisierungsmittel enthalten, sind Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

Unabhängig davon, ob Zubereitungen (C) und/oder Zubereitung (B) und/oder weitere Zubereitungen Alkalisierungsmittel enthalten, sind, wenn Alkalisierungsmittel zum Einsatz kommen, solche Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

Zur weiteren Steigerung der Aufhellleistung kann Zubereitung (C) zusätzlich als Bleichverstärker eine Silicium-haltige Verbindung zugesetzt werden. Diese wird bevorzugt ausgewählt aus der Gruppe, die gebildet wird aus Kieselsäure, Alkalimetallsilicaten und Erdalkalimetallsilicaten.

Obwohl bereits geringe Mengen der Silicium-haltigen Verbindungen die Aufhellleistung erhöhen, kann es erfindungsgemäß bevorzugt sein, die Silicium-haltigen Verbindungen in Mengen von 0,05 Gew.-% bis 50 Gew.-%, bevorzugt in Mengen von 0,5 Gew.-% bis 30 Gew.-% und besonders bevorzugt in Mengen von 1,0 Gew.-% bis 25 Gew.-%, jeweils bezogen auf das Gesamtgewicht der Zubereitung (C), einzusetzen.

Als Silicium-haltige Verbindungen werden insbesondere Alkalimetallsilicate in Form von Wasserglas eingesetzt. Unter Wasserglas ist dabei eine Verbindung zu verstehen, die aus einem Silicat der Formel (SiO₂)ₙ(Na₂O)ₘ(K₂O)ₚ gebildet wird, wobei n für eine positive rationale Zahl und m und p unabhängig voneinander für eine positive rationale Zahl oder für 0 stehen, mit den Maßgaben, dass mindestens einer der Parameter m oder p von 0 verschieden ist und das Verhältnis zwischen n und der Summe aus m und p zwischen 1:1 und 4:1 liegt.

Neben den durch die Summenformel beschriebenen Komponenten können die Wassergläser in geringen Mengen noch weitere Zusatzstoffe, wie Phosphate oder Magnesiumsalze, enthalten.

Erfindungsgemäß besonders bevorzugte Wassergläser werden unter anderem unter den Bezeichnungen Ferrosil^{®} 119, Natronwasserglas 40/42, Portil^{®} A, Portil^{®} AW und Portil^{®} W und Britesil^{®} C20 vertrieben.

Weiterhin können als Silicium-haltige Verbindungen insbesondere Kieselsäuren eingesetzt werden, die auch als Silica oder Kieselgel vertrieben werden. Bevorzugt ist dabei ein Kieselgel, welches unter dem Handelsnamen Aerosil 200 (INCI-Bezeichnung: Silica) vertrieben wird.

Weiterhin können die Aufhell- bzw. Blondiermittel zur Verstärkung der Blondierwirkung noch zusätzliche Bleichkraftverstärker enthalten. Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker vom Typ der Kohlensäurederivate können erfindungsgemäß bevorzugt Carbonatsalze bzw. Hydrogencarbonatsalze eingesetzt werden. Diese sind bevorzugt ausgewählt aus der Gruppe der Ammonium-, Alkalimetall- (insbesondere Na und K) sowie Erdalkalimetall-(insbesondere Mg und Ca), -carbonatsalze bzw. -hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese besonders bevorzugten Salze können allein oder in deren Mischungen von mindestens zwei Vertretern als Bleichverstärker verwendet werden. Weiterhin sind Alkylcarbonate, -carbamate, Silylcarbonate sowie -carbamate geeignete Bleichverstärker.

Weiterhin erfindungsgemäß einsetzbare Bleichkraftverstärker können aus stickstoffhaltigen, gegebenenfalls kationischen Heterocyclen, insbesondere Imidazol, ausgewählt werden.

Besonders bevorzugte stickstoffhaltige, heterocyclische Bleichkraftverstärker sind die quaternierten Kationen von Pyridinen und 3,4-Dihydroisochinolinen, wie Salze des 4-Acetyl-1-methylpyridiniums, insbesondere 4-Acetyl-1-methylpyridinium-p-toluolsulfonat, Salze des 2-Acetyl-1-methylpyridiniums, insbesondere 2-Acetyl-1-methylpyridinium-p-toluolsulfonat, sowie Salze des N-Methyl-3,4-dihydroisochinoliniums, insbesondere N-Methyl-3,4-dihydroisochinolinium-p-toluol-sulfonat.

Weiterhin erfindungsgemäß einsetzbarer Bleichkraftverstärker ist Harnstoff.

Bleichkraftverstärker können in Zubereitung (A) und/oder Zubereitung (B) und/oder gegebenenfalls Zubereitung (C) und/oder gegebenenfalls weiteren Zubereitungen enthalten sein. Dabei können die Bleichkraftverstärker entweder in nur einer der Zubereitungen oder in zwei oder mehr der Zubereitungen enthalten sein. Hydrolyseempfindliche Bleichkraftverstärker können bevorzugt in der pulverförmigen Zubereitung (A) eingesetzt werden. Unabhängig davon, ob Bleichkraftverstärker in Zubereitung (A) und/oder Zubereitung (B) und/oder Zubereitung (C) und/oder weiteren Zubereitungen eingesetzt werden, sind diese, wenn Bleichkraftverstärker zum Einsatz kommen, bevorzugt in Mengen von 0,5 bis 30 Gew.-%, insbesondere in Mengen von 2 bis 20 Gew.-%, jeweils bezogen auf das Gesamtgewicht der fertig angemischten Blondierzubereitung, enthalten.

Weiterhin können die Aufhell- bzw. Blondiermittel zur Mattierung von unerwünschten Restfarbeindrücken, insbesondere im rötlichen oder bläulichen Bereich, bestimmte, direktziehende Farbstoffe der komplementären Farben enthalten. Dabei handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe sind als anionische, kationische und nichtionische direktziehende Farbstoffe bekannt. Die direktziehenden Farbstoffe werden jeweils bevorzugt in einer Menge von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Ganz besonders bevorzugt sind Mittel, die mindestens eine Kombination aus Tetrabromphenolblau und Acid Red 92 enthalten.

Weiterhin hat es sich als vorteilhaft erweisen, wenn die anwendungsbereiten Mittel mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Diese können unterschiedlichen chemischen Gruppen angehören. Vorzugsweise werden einzeln oder im Gemisch miteinander eingesetzt. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide, insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate; Lichtschutzmittel, wie derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, B₃, B₅, B₆, C, E, F und H; Pflanzenextrakte; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Besonders bevorzugt gemäß der Erfindung sind solche Wirk-, Hilfs- und Zusatzstoffe, die in Kombination mit dem erfindungsgemäßen Mittel zu einer transparenten Anwendungsmischung führen.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Ein zweiter Gegenstand der Erfindung ist ein Verfahren zur Farbveränderung keratinischer Fasern, dadurch gekennzeichnet, dass mindestens zwei getrennt voneinander verpackte Zubereitungen (A) und (B), von welchen Zubereitung (A) mindestens ein Persulfat enthält und frei von Xanthan ist, und Zubereitung (B) mindestens ein Oxidationsmittel und Xanthan enthält, zu einer Anwendungsmischung vermischt werden, diese auf die Fasern aufgetragen wird und nach einer Einwirkungszeit wieder abgespült wird.

Die anwendungsbereiten Mittel werden unmittelbar vor der Anwendung auf dem Haar durch Mischen der zwei Zubereitungen (A) und (B) und gegebenenfalls einer dritten Zubereitung (C) und/oder weiteren Zubereitungen hergestellt. Bei anwendungsbereiten Mitteln, die aus mehr als zwei Zubereitungen zu einer fertigen Anwendungsmischung vermischt werden, kann es unerheblich sein, ob zunächst zwei Zubereitungen miteinander vermischt werden und anschließend die dritte Zubereitung zugegeben und untergemischt wird, oder ob alle Zubereitungen gemeinsam zusammengeführt und anschließend vermischt werden. Das Vermischen kann durch Verrühren in einer Schale oder einem Becher erfolgen oder durch Schütteln in einem verschließbaren Behälter.

Der Begriff "unmittelbar" ist dabei als Zeitraum von wenigen Sekunden bis eine Stunde, vorzugsweise bis 30 min, insbesondere bis 15 min zu verstehen.

Die erfindungsgemäßen Mittel werden in einem Verfahren zum Aufhellen von keratinischen Fasern, insbesondere menschlichen Haaren, angewendet, bei dem das Mittel auf die keratinhaltigen Fasern aufgebracht, bei einer Temperatur von Raumtemperatur bis 45 °C für eine Einwirkdauer von 10 bis 60 Minuten auf der Faser belassen und anschließend mit Wasser wieder ausgespült oder mit einem Shampoo ausgewaschen wird.

Bevorzugt beträgt die Einwirkzeit der anwendungsbereiten Aufhellmittel 10 bis 60 min, insbesondere 15 bis 50 min, besonders bevorzugt 20 bis 45 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie mit Hilfe eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Bevorzugt liegt die Temperatur während der Einwirkzeit zwischen 20°C und 40°C, insbesondere zwischen 25°C und 38°C. Die Aufhellmittel ergeben bereits bei physiologisch verträglichen Temperaturen von unter 45°C gute Blondier- und Aufhellergebnisse.

Nach Ende der Einwirkzeit wird die verbleibende Aufhellzubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark tensid-haltigen Träger besitzt.

Ein bevorzugtes Verfahren ist dadurch gekennzeichnet, dass die Kontrolle des Aufhellgrades der keratinischen Faser während der Einwirkzeit ohne Entfernen der Anwendungsmischung von der Faser visuell erfolgt. Dazu wird ein anwendungsbereites, transparentes Mittel des ersten Erfindungsgegenstands auf menschliches Haar aufgetragen, und der Aufhellprozess während der Einwirkzeit ein oder mehrmals durch visuelle Kontrolle, ohne Abtragen des Mittels von der Faser, beurteilt. Somit ist eine vereinfachte und permanente Kontrolle des Entfärbevorgangs gewährleistet.

"Transparent" im Sinne der Erfindung sind Zubereitungen, die bei Auftragung einer gleichmäßigen Schicht dieser Zubereitungen von 1 bis 3 mm Dicke auf einen Untergrund klar sind und so durchblickt werden können, dass das menschliche Auge die Farbe des Untergrunds ohne Trübung erkennen und beurteilen kann. Transparenz kann vom Fachmann weiterhin durch technische Methoden gemessen werden. "Transparent" im Sinne der Erfindung sind daher auch Zubereitungen, die bei photometrischen Messungen mit einem Methrom Photometer 662 bei 25°C Transmissionen von mindestens 70%, insbesondere mindestens 80%, erreichen.

Die bevorzugten Ausführungsformen des ersten Erfindungsgegenstands gelten mutatis mutandis auch für den zweiten Erfindungsgegenstand.

Ein dritter Gegenstand der vorliegenden Erfindung ist ein Verfahren zur Herstellung einer fließfähigen Oxidationsmittelzubereitung, die mindestens ein Oxidationsmittel enthält, dadurch gekennzeichnet, dass in die Zubereitung pulverförmiges Xanthan Gum eingerührt wird, welches die Eigenschaft aufweist, dass 1 Gew.-% des Xanthan Gum in 1 Gew.-% wässriger KCl Lösung eine Viskosität von 800-1300 mPa·s bei Messungen mit einem Rotationsviskosimeter von Brookfield, Spindelgröße 4, bei 25°C und 4 Upm zeigt.

Vorzugsweise liegt das verwendete Xanthan Gum vor der Quellung in pulverförmigem Zustand vor und ist dadurch gekennzeichnet, dass das pulverförmige Xanthan eine Partikelgröße zwischen 160 µm und 200 µm aufweist.

Die Konfektionierung der erfindungsgemäßen Aufhellmittel unterliegt prinzipiell keinerlei Beschränkungen. Um dem Anwender die Komponenten des anwendungsbereiten Mittels möglichst komfortabel anzubieten, ist es sinnvoll, die einzelnen Zubereitungen in einer Verpackungseinheit gemeinsam zu vertreiben.

Ein vierter Gegenstand der vorliegenden Erfindung ist daher eine Mehrkomponentenverpackungseinheit (Kit-of-Parts) zur Aufhellung und/oder Blondierung von keratinischen Fasern, umfassend in jeweils getrennt voneinander konfektionierten Containern
a) mindestens eine pulverförmige Zubereitung (A), enthaltend mindestens ein Persulfat,
b) mindestens eine fließfähige Zubereitung (B), enthaltend mindestens ein Oxidationsmittel
c) gegebenenfalls eine weitere Zubereitung (C), enthaltend mindestens ein Alkalisierungsmittel und mindestens ein natürliches Polymer
d) und /oder gegebenenfalls weitere Zubereitungen,
wobei Zubereitung (B) Xanthan enthält und Zubereitung (A) frei von Xanthan ist.

Die Komponenten der Mehrverpackungseinheit werden getrennt voneinander in räumlich unterschiedlichen Behältern oder Containern konfektioniert. Der Begriff "Behälter" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff Behälter umfasst daher, ohne darauf beschränkt zu sein, das Innere einer Tube, eines Beutels oder Sackes, eines Kanisters, einer Dose, einer Wanne, einer Flasche, eines Glases oder eines Paketes, eines Kartons, einer Box, eines Umschlages oder eines anderen Behältnisses. Die Behälter können mit einer wiederverschließbaren Öffnung wie einem Schraubverschluss versehen sein. Dies kann insbesondere dann von Vorteil sein, wenn mehrere Mittel vor der Anwendung durch Schütteln innig miteinander vermischt werden sollten.

Die Komponenten der Aufhellzubereitung können in einem Doppelkammer-Behälter mit getrennter oder gemeinsamer Öffnung enthalten sein. Es ist jedoch bevorzugt, sie auf verschiedene Behälter aufzuteilen und den Verbraucher anzuleiten, sie vor der Anwendung miteinander zu mischen.

Bevorzugt enthält die Mehrkomponentenverpackungseinheit (Kit-of-Parts) zusätzlich eine Gebrauchsanleitung. Die Gebrauchsanleitung enthält insbesondere Informationen, Erläuterungen und gegebenenfalls Illustrationen für den Verbraucher zur Anwendung der Mittel aus den Behältern der Verpackungseinheit in einem Verfahren gemäß dem zweiten Erfindungsgegenstand. Darüber hinaus kann es bevorzugt sein, wenn weiterhin eine Anmischhilfe, wie beispielsweise eine Schale, eine Applikationshilfe, wie beispielsweise ein Kamm oder ein Pinsel, und/oder eine persönliche Schutzausrüstung, wie beispielsweise Einweg-Handschuhe dem Kit beigefügt sind.

Bezüglich bevorzugter Ausführungsformen der erfindungsgemäßen Mehrkomponentenverpackungseinheit gelten mutatis mutandis die Ausführungsformen der vorangegangenen Erfindungsgegenstände.

### Beispiele

### Beispiel 1: Zusammensetzung Zubereitung (A) für Blondiermittel aus zwei Zubereitungen

| Beschreibung | A1 | A2 | A3 | A4 | A5 | A6 | A7 | A8 |
|---|---|---|---|---|---|---|---|---|
| EDTA Na2 | 1,51 | 1 | 1,51 | 1,51 | 1,51 | 1,51 | 1,51 | 1,51 |
| Natriumhexametaphosphat | 0,3 | 0,3 | 0,3 | 0,3 | 0,3 | 0,3 | 0,3 | 0,3 |
| Aerosil 200 | 3,02 | 1 | 3 | 3 | 3 | 3 | 3 | 3 |
| Natriummetasilikat FE wasserfrei | 26 | 26 | 26 | 26 | 26 | 26 | 26 | 26 |
| Calciumstearat | 0,9 | 0,9 | 0,9 | 0,9 | 0,9 | 0,9 | 0,9 | 0,9 |
| Rohagit S hv | 3,02 | 4,05 | 1 | | | 2,5 | 3,5 | |
| Cekol 50000 | 5 | 6,5 | 2,5 | 2,5 | 2,5 | 1 | | |
| Ammoniumpersulfat + 0,5 % Kieselsäure | 19,9 | 19,9 | 19,9 | 19,9 | 19,9 | 19,9 | 19,9 | 19,9 |
| Kaliumpersulfat | 7,4 | 7,4 | 11,94 | 11,94 | 11,94 | 11,94 | 11,94 | 15,44 |
| Natriumpersulfat | 24,9 | 24,9 | 24,9 | 24,9 | 24,9 | 24,9 | 24,9 | 24,9 |
| Paraffinum Liquidum | 7,85 | 7,85 | 7,85 | 7,85 | 7,85 | 7,85 | 7,85 | 7,85 |
| Parfum ROSEX FINE INC 637510 | 0,2 | 0,2 | 0,2 | 0,2 | 0,2 | 0,2 | 0,2 | 0,2 |
| LAPONITE XLG | | | | 1 | | | | |
| LAPONITE XLS | | | | | 1 | | | |

| | | | | | | | | |
|---|---|---|---|---|---|---|---|---|
| *eingesetzte Rohstoffe: Aerosil 200 (INCI Bezeichnung: Silica (Evonik Degussa)), Rohagit S hv (INCI Bezeichnung: Acrylates Copolymer (Evonik Röhm)), Cekol 50000 (INCI Bezeichnung: Cellulose Gummi (CP Kelco)), LAPONITE XLG (INCI Bezeichnung: Natrium-Magnesium-Silicat (Roockwood Additives)), LAPONITE XLS (INCI Bezeichnung: Natrium-Magnesium-Silicat und Tetranatriumpyrophosphat (Roockwood Additives)), | | | | | | | | |

### Beispiel 2: Zusammensetzung Zubereitung (B) für Blondiermittel aus zwei Zubereitungen

| Beschreibung | B1 | B2 | B3 | B4 | B5 |
|---|---|---|---|---|---|
| Wasser, vollentsalzt | ad 100 | ad 100 | ad 100 | ad 100 | ad 100 |
| Natronlauge 45 % techn. | 0,73 | 0,73 | 0,73 | 0,73 | 0,73 |
| Dipicolinsäure | 0,1 | 0,1 | 0,1 | 0,1 | 0,1 |
| Dinatrium pyrophosphat | 0,03 | 0,03 | 0,03 | 0,03 | 0,03 |
| HEDP 60% | 1,5 | 1,5 | 1,5 | 1,5 | 1,5 |
| Keltrol CG-SFT | 2 | | 1,5 | 1 | 0,5 |
| Propandiol-1,2 | 4 | 2 | 4 | 4 | 4 |
| Wasserstoffperoxid 50 % | 18 | 18 | 18 | 18 | 18 |
| Xanthan FN | | 1 | | | |

| | | | | | |
|---|---|---|---|---|---|
| *eingesetzte Rohstoffe: Keltrol CG-SFT (INCI Bezeichnung: Xanthan Gum (CP Kelco)), Xanthan FN (INCI Bezeichnung: Xanthan-Gum (Jungbunzlauer)) | | | | | |

Bei den Zubereitungen B1 bis B5 handelt es sich um transparente Gele.

### Beispiel 3: Zusammensetzung Zubereitung (A) für Blondiermittel aus drei Zubereitungen

| Bezeichnung | A9 | A10 | A11 |
|---|---|---|---|
| Kaliumpersulfat | 98,6 | 42,8 | 60 |
| Aerosil 200 | 1,4 | - | - |
| Ammoniumpersulfat + 4 % Kieselsäure | - | 13 | 18,2 |
| Natriumpersulfat | - | 15,6 | 21,8 |
| Britesil C 20 | - | 28 | - |

| | | | |
|---|---|---|---|
| *eingesetzte Rohstoffe: Aerosil 200 (INCI Bezeichnung: Silica (Evonik Degussa)), Britesil C 20 (INCI Bezeichnung: Natriumsilicat (PQ Europe)) | | | |

### Beispiel 4: Zusammensetzung Zubereitung (B) für Blondiermittel aus drei Zubereitungen. Zubereitungen B10 und B12 sind nicht erfindungsgemäß nach Anspruch 1.

| Bezeichnung | B6 | B7 | B8 | B9 | B10 | B11 | B12 |
|---|---|---|---|---|---|---|---|
| Wasser, vollentsalzt | ad 100 | ad 100 | ad 100 | ad 100 | ad 100 | ad 100 | ad 100 |
| Natronlauge 45 % techn. | 0,73 | 0,73 | 0,73 | 0,73 | 0,73 | 0,73 | 0,73 |
| Dipicolinsäure | 0,1 | 0,1 | 0,1 | 0,1 | 0,1 | 0,1 | 0,1 |
| Dinatriumpyrophosphat | 0,03 | 0,03 | 0,03 | 0,03 | 0,03 | 0,03 | 0,03 |
| HEDP 60% | 1,5 | 1,5 | 1,5 | 1,5 | 1,5 | 1,5 | 1,5 |
| Keltrol CG-SFT | 2 | 1,5 | 1 | 0,5 | | 0,5 | |
| Propandiol-1,2 | 4 | 4 | 4 | 4 | | 4 | |
| Wasserstoffperoxid 50 % | 18 | 18 | 18 | 18 | 18 | 18 | 18 |
| Mergital CS 50 A | | | | | | 10 | 10 |

| | | | | | | | |
|---|---|---|---|---|---|---|---|
| *eingesetzte Rohstoffe: Keltrol CG-SFT (INCI Bezeichnung: Xanthan Gum (CP Kelco)), Mergital CS 50 A (INCI Bezeichnung: Ceteareth-50 (Cognis)) | | | | | | | |

### Beispiel 5: Zusammensetzung Zubereitung (C) für Blondiermittel aus drei Zubereitungen

| Bezeichnung | C1 | C2 | C3 |
|---|---|---|---|
| Wasser, vollentsalzt | ad 100 | ad 100 | ad 100 |
| Monoethanolamin | 8 | 8 | 8 |
| Ascorbinsäure | 0,4 | 0,4 | 0,4 |
| Natriumsulfit wasserfrei A 96% MIN | 0,4 | 0,4 | 0,4 |
| HEDP 60% | 0,2 | 0,2 | 0,2 |
| Keltrol CG-SFT | 2 | 4 | 6 |
| Propandiol-1,2 | 4 | 6 | 8 |
| Natronwasserglas | 0,5 | 0,5 | 0,5 |

| | | | |
|---|---|---|---|
| *eingesetzte Rohstoffe: Keltrol CG-SFT (INCI Bezeichnung: Xanthan Gum (CP Kelco)) | | | |

### Beispiele Anwendungszubereitungen

### Beispiel 6: Blondiermittel aus zwei Zubereitungen

Die einzelnen Zubereitungen wurden in den angegebenen Mengen miteinander Vermischt und die Viskosität der fertigen Anwendungszubereitungen mit einem Rotationsviskosimeter von Brookfield, Spindelgröße 4, bei 25°C und 4 Upm gemessen.

| Bezeichnung | AZ 1 | AZ 2 | AZ 3 | AZ 4 | AZ 5 | AZ 6 | AZ 7 | AZ 8 |
|---|---|---|---|---|---|---|---|---|
| B5 | 80g | | | | | | | |
| B4 | | 80g | | | 80g | | | |
| B3 | | | 80g | | | 80g | | |
| B1 | | | | 80g | | | 80g | |
| A8 | 20g | 20g | 20g | 20g | | | | |
| A1 | | | | | 20g | 20g | 20g | |
| Vergl.* | | | | | | | | 120g* |
| mVisk Sp5/4U pm | 16300 mPas | 33100 mPas | 41500 mPas | 69700 mPas | 81300 mPas | 112000 mPas | 129000 mPas | 90750 mPas |
| Bewertu ng | klares Gel | klares Gel | klares Gel | klares Gel | klares Gel | klares Gel | klares Gel | weiße Emulsion |

| | | | | | | | | |
|---|---|---|---|---|---|---|---|---|
| * Vergleich mit einem auf dem Markt befindlichen Produkt: 80g Clynol Viton (9% H2O2) ergibt zusammen mit 40g Viton super lightening white eine weiße Emulsion mit einer Viskosität von 90750 mPas. | | | | | | | | |

### Beispiel 7: Blondiermittel aus drei Zubereitungen

| Bezeichnung | AZ 9 | AZ 10 | AZ 11 | AZ 12 | AZ 13 |
|---|---|---|---|---|---|
| B10 | 50g | 50g | 40g | 50g | |
| B9 | | | | | 50g |
| C2 | 50g | 50g | 40g | 50g | 50g |
| A9 | 8,5g | | | | 8,5g |
| A10 | | 20g | | | |
| A11 | | | 14g | | |
| mVisk | 35400 | 38000 | 37200 | 37100 | 46600 |
| Sp5/4Upm | mPas | mPas | mPas | mPas | mPas |
| Bewertung | klares Gel | klares Gel | klares Gel | klares Gel | klares Gel |

Die einzelnen Zubereitungen wurden in den angegebenen Mengen miteinander vermischt und die Viskosität der fertigen Anwendungszubereitungen mit einem Rotationsviskosimeter von Brookfield, Spindelgröße 5, bei 25°C und 4 Upm gemessen.

Anwendungszubereitungen AZ 9, AZ 10, AZ 11 und AZ 12 sind nicht Zubereitungen gemäß der Erfindung nach Anspruch 1. AZ 9 zeigt ein Beispiel für eine Anwendungszubereitung ohne Ammoniak.

## Patentansprüche

1. Mittel zum Aufhellen keratinischer Fasern, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
i. Zubereitung (A) mindestens ein Persulfat enthält und
ii. Zubereitung (B) fließfähig ist und mindestens ein Oxidationsmittel enthält,
**dadurch gekennzeichnet, dass** Zubereitung (B) Xanthan enthält und Zubereitung (A) frei von Xanthan ist.

2. Mittel nach Anspruch 1, **dadurch gekennzeichnet, dass** Zubereitung (A) mindestens ein Persulfat, ausgewählt aus Ammoniumperoxodisulfat und/oder Kaliumperoxodisulfat und/oder Natriumperoxodisulfat, enthält.

3. Mittel nach einem der Ansprüche 1 bis 2, **dadurch gekennzeichnet, dass** Zubereitung (B) bezogen auf ihr Gewicht Wasserstoffperoxid in Mengen von 0,5 bis 30 Gew.-%, vorzugsweise von 1 bis 20 Gew.-%, besonders bevorzugt von 5 bis 15 Gew.-% und insbesondere bevorzugt von 6 bis 12 Gew.-%, enthält.

4. Mittel nach einem der Ansprüche 1 bis 3, enthaltend mindestens eine weitere getrennt verpackte Zubereitung (C), **dadurch gekennzeichnet, dass** Zubereitung (C) mindestens ein Alkalisierungsmittel und Xanthan enthält.

5. Mittel nach einem der Ansprüche 1 bis 4, **dadurch gekennzeichnet, dass** die fertig vermischte Anwendungsmischung, bezogen auf ihr Gewicht, Xanthan in Mengen von 0,6 bis 5 Gew.-%, bevorzugt von 1,0 bis 3,5 Gew.-% und besonders bevorzugt 1,5 bis 2,5 Gew.-% enthält.

6. Mittel nach einem der Ansprüche 1 bis 5, **dadurch gekennzeichnet, dass** die fertig angemischte Anwendungszubereitung eine Visokitäten von 10.000 mPa•s bis 50.000 mPa•s, bevorzugt von 18.000 mPa•s bis 30.000 mPa•s bei Messungen mit einem Rotationsviskosimeter von Brookfield, Spindelgröße 5, bei 25°C und 4 Upm aufweist.

7. Verfahren zur Farbveränderung keratinischer Fasern, **dadurch gekennzeichnet, dass** mindestens zwei getrennt voneinander verpackte Zubereitungen (A) und (B), von welchen Zubereitung (A) mindestens ein Persulfat enthält und frei von Xanthan ist, und Zubereitung (B) mindestens ein Oxidationsmittel und Xanthan enthält, zu einer Anwendungsmischung vermischt werden, diese auf die Fasern aufgetragen wird und nach einer Einwirkungszeit wieder abgespült wird.

8. Verfahren zur Farbveränderung keratinischer Fasern nach Anspruch 7, **dadurch gekennzeichnet, dass** die Kontrolle des Aufhellgrades der keratinischen Faser während der Einwirkzeit ohne Entfernen der Anwendungsmischung von der Faser visuell erfolgt.

## Claims

1. Agents for lightening keratinic fibers, containing at least two separately packaged preparations (A) and (B) and optionally one further preparation (C) that is packaged separately from (A) and (B), which preparations are mixed immediately before application to produce an application mixture,
i. preparation (A) containing at least one persulfate, and
ii. preparation (B) being flowable and containing at least one oxidizing agent,
**characterized in that** preparation (B) contains xanthan and preparation (A) is free of xanthan.

2. The agents according to claim 1, **characterized in that** preparation (A) contains at least one persulfate selected from ammonium peroxodisulfate and/or potassium peroxodisulfate and/or sodium peroxodisulfate.

3. The agents according to one of claims 1 to 2, **characterized in that** preparation (B) contains, based on its weight, hydrogen peroxide in amounts of from 0.5 to 30 wt.%, preferably of from 1 to 20 wt.%, particularly preferably of from 5 to 15 wt.% and more particularly preferably of from 6 to 12 wt.%.

4. The agents according to one of claims 1 to 3, containing at least one further separately packaged preparation (C), **characterized in that** preparation (C) contains at least one alkalizing agent and xanthan.

5. The agents according to one of claims 1 to 4, **characterized in that** the ready-mixed application mixture contains, based on its weight, xanthan in amounts of from 0.6 to 5 wt.%, preferably of from 1.0 to 3.5 wt.% and particularly preferably of from 1.5 to 2.5 wt.%.

6. The agents according to one of claims 1 to 5, **characterized in that** the ready-mixed application preparation has a viscosity of from 10,000 mPa·s to 50,000 mPa·s, preferably of from 18,000 mPa·s to 30,000 mPa·s, in measurements using a Brookfield rotational viscometer, spindle size 5, at 25°C and 4 rpm.

7. A method for changing the color of keratinic fibers, **characterized in that** at least two separately packaged preparations (A) and (B), of which preparation (A) contains at least one persulfate and is free of xanthan, and preparation (B) contains at least one oxidizing agent and xanthan, are mixed to produce an application mixture, said mixture is applied to the fibers and is rinsed out again after a contact time.

8. The method for changing the color of keratinic fibers according to claim 7, **characterized in that** monitoring the degree of lightening of the keratinic fibers during the contact time takes place visually without removing the application mixture from the fibers.

## Revendications

1. Produit d'éclaircissement de fibres de kératine, contenant au moins deux préparations en emballages séparés (A) et (B) ainsi que, éventuellement, une autre préparation (C) emballée séparément de (A) et (B), qu'on mélange immédiatement avant utilisation en un mélange prêt à l'emploi :
i. la préparation (A) contenant au moins un persulfate, et
ii. la préparation (B) pouvant s'écouler et contenant au moins un oxydant,
**caractérisé en ce que** la préparation (B) contient du xanthane et la préparation (A) ne contient pas de xanthane.

2. Produit selon la revendication 1, **caractérisé en ce que** la préparation (A) contient au moins un persulfate choisi parmi le peroxodisulfate d'ammonium et/ou le peroxodisulfate de potassium et/ou le peroxodisulfate de sodium.

3. Produit selon une des revendications 1 ou 2, **caractérisé en ce que** la préparation (B), rapporté à son poids, contient du peroxyde d'hydrogène à hauteur de 0,5 à 30 % en poids, de préférence de 1 à 20 % en poids, particulièrement préférentiellement de 5 à 15 % en poids et en particulier de 6 à 12 % en poids.

4. Produit selon une des revendications 1 à 3, contenant au moins une autre préparation (C) emballée séparément, **caractérisé en ce que** la préparation (C) contient au moins une base et du xanthane.

5. Produit selon une des revendications 1 à 4, **caractérisé en ce que** le mélange prêt à l'emploi, fraîchement mélangé, contient, rapporté à son poids, du xanthane à hauteur de 0,6 à 5 % en poids, de préférence de 1,0 à 3,5 % en poids, et particulièrement préférentiellement de 1,5 à 2,5 % en poids.

6. Produit selon une des revendications 1 à 5, **caractérisé en ce que** le mélange prêt à l'emploi, fraîchement mélangé présente une viscosité de 10 000 à 50 000 mPa.s, de préférence de 18 000 à 30 000 mPa.s mesuré dans un viscosimètre à rotation Brookfield, taille de rotor 5, à 25°C et 4 tr/min.

7. Procédé de coloration de fibres de kératine, **caractérisé en ce qu'**au moins deux préparations emballées séparément (A) et (B), parmi lesquelles la préparation (A) contient au moins un persulfate et ne contient pas de xanthane, et la préparation (B) contient au moins un oxydant et du xanthane, sont mélangées en un mélange prêt à l'emploi qu'on applique sur les cheveux puis qu'on rince près une durée d'action.

8. Procédé de coloration de fibres de kératine selon la revendication 7, **caractérisé en ce qu'**on contrôle visuellement le degré d'éclaircissement des fibres de kératine pendant la durée d'action sans éliminer le mélange prêt à l'emploi des fibres.
